# EUROPEAN PATENT SPECIFICATION

(11) **EP 1 173 153 B1**
(45) Date of publication and mention of the grant of the patent: **30.11.2005**
(21) Application number: 00919874.8
(22) Date of filing: 29.03.2000
(51) Int. Cl.: A61K 9/48, A61K 9/66

(54) **ORAL PHARMACEUTICAL COMPOSITIONS CONTAINING LONG-CHAIN TRIGLYCERIDES AND LIPOPHILIC SURFACTANTS**
ORALE PHARMAZEUTISCHE ZUSAMMENSETZUNGEN, DIE LANGKETTIGE TRIGLYCERIDE UND LIPOPHILE TENSIDE ENTHALTEN
COMPOSITIONS PHARMACEUTIQUES ORALES RENFERMANT DES TRIGLYCERIDES A LONGUE CHAINE ET DES TENSIOACTIFS LIPOPHILES

(30) Priority: 01.04.1999 GB 9907715
(43) Date of publication of application: 23.01.2002
(73) Proprietor: R.P. SCHERER CORPORATION, Basking Ridge, New Jersey 07920 (US)
(72) Inventor: PERRY, Elizabeth A., Highworth, Swindon SN6 7BJ (GB); CHANDLER, Susan, Wiltshire Marlsborough (GB); FERDINANDO, Josephine Joan Christine, Chippenham, Wiltshire SN15 2XM (GB); DE NIJS, Henrik, NL-5343 XC Oss (NL)
(74) Representative: Pearce, Anthony Richmond
(86) International application number: PCT/US2000/008426
(87) International publication number: WO 2000/059482

(56) References cited:
- WO-A-00/59512
- WO-A-95/24893
- WO-A-97/40823
- US-A- 5 645 856
- NOGUCHI T ET AL: "The effect of drug lipophilicity and lipid vehicles on the lymphatic absorption of various testosterone esters" INTERNATIONAL JOURNAL OF PHARMACEUTICS, vol. 24, no. 2-3, 1985, pages 173-184, XP001050529 ISSN: 0378-5173

## Description

### Field of the Invention

The invention relates to pharmaceutical compositions for oral administration. In particular, the invention relates to liquid pharmaceutical compositions suitable for softgel encapsulation.

### Background of the Invention

A number of drugs are known to require formulation in the presence of fatty acids, such as oleic acid, to provide optimum conditions for bioavailability. For example, long-chain fatty acids can be predisposed for lymphatic absorption, and therefore, are useful in pharmaceutical formulations in which the lymphatic system is the desired target site for the active ingredient. One of the problems associated with the formulations containing fatty acids is that chemical instability can arise due to their acidic nature and the presence of reactive carboxyl groups. Esterification can occur with drug molecules containing alcohol groups or transesterification of ester molecules.

In the past, this problem has been solved by continuing to use free fatty acids in formulations which are encapsulated in softgel capsules and storing the capsules under refrigerated conditions to reduce the rate of reaction between the drug and fatty acids. However, where the drug is not sufficiently soluble in the formulation, cold storage methods result in crystallization which in turn necessitates equilibrating the capsules at room temperature to ensure crystal dissolution prior to consumption. Accordingly, complex storage regimens are required throughout the supply chain for the use of such formulations.

Lacy et al. in Patent Cooperation Treaty WO 95/24893 published September 21, 1995 discloses a carrier system for a hydrophobic drug which comprises a digestible oil and a pharmaceutically acceptable surfactant for dispersing the oil *in vivo* upon administration of the carrier system, the surfactant comprising a hydrophilic surfactant component which substantially inhibits the lipolysis of the digestible oil, and a lipophilic surfactant component which substantially reduces the inhibitory effect of the hydrophilic surfactant component. Suitable digestible oils are complete or partial esters of medium chain (C₈-C₁₂) or long-chain (C₁₄ - C₂₂) fatty acids with low molecular weight (up to C₆) mono-, di- or polyhydric alcohols. Medium chain length triglycerides or long chain tri- and diglyceride mixtures which may contain monoglycerides are particularly preferred. Fractionated coconut oil is a preferred oil.

The lipophilic surfactants used include fatty acids; mono- and/or diglycerides of fatty acids; acetic, succinic, lactic, citric and/or tartaric esters of mono and/or diglycerides of fatty acids; propylene glycol mono- and/or di-esters of fatty acids; polyglycerol esters of fatty acids; castor oil ethoxylates; acid and ester ethoxylates; and sorbitan esters of fatty acids.

The hydrophilic surfactants used have a hydrophilic/lipophilic balance (HLB) value greater than 10 and include phospholipid; polyoxyethylene sorbitan fatty acid derivatives; castor oil or hydrogenated castor oil ethoxylates; fatty acid ethoxylates; alcohol ethoxylates; polyoxyethylene, polyoxypropylene co-polymers and block co-polymers; anionic surfactants and alkylphenol surfactants.

The formulations may contain ethanol as a co-solvent and formulations containing up to 15 % by weight ethanol are disclosed.

Perry et al. in Patent Cooperation Treaty WO 97/40823 published November 6, 1997 discloses a pharmaceutical composition comprising a hydrophobic drug; a digestible oil selected from triglycerides or propylene glycol esters of medium chain length (C₈-C₁₂) and/or long chain length (C₁₃-C₂₂) fatty acids; propylene glycol monolaurate (lauroglycol), a lipophilic surfactant which comprises a glyceride of a C₅ to C₁₀ fatty acid; and a hydrophilic surfactant which is a polyoxyethylene hydrogenated castor oil, wherein the digestible oil is present in an amount in the range from 3.0 to 12.0% by weight of the composition and the weight ratio of hydrophilic surfactant to lipophilic surfactant is in the range of 1:1.5 to 1:2.5. The formations may contain ethanol as a co-solvent for the drug and formulations containing 15-25% by weight ethanol are disclosed.

There still exists the need for pharmaceutical compositions having improved stability which promote systemic absorption

### Summary of the Invention

The invention discloses a liquid pharmaceutical composition having a drug dissolved in a liquid vehicle, said liquid vehicle comprising:
a) a glyceride of a long chain fatty acid; and
b) a lipophilic surfactant having an HLB of less than 10;
characterised in that the drug is testosterone undecanoate, said liquid vehicle contains less than 1% by weight of free fatty acids, and said glyceride of a long chain fatty acid is a triglyceride of a fatty acid having 14 to 22 carbon atoms.

The composition according to the invention is stable and promotes systemic absorption of testosterone undecanoate (also referred to as TU) which is susceptible to the adverse effects of carboxyl groups in carrier ingredients.

Preferably, the triglyceride of a fatty acid having 14 to 22 carbon atoms is present in an amount from 15 % to 70 % by weight, more preferably from 15% to 60 % by weight, and most preferably from 40 % to 60 % by weight, of said liquid vehicle; and the lipophilic surfactant having an HLB of less than 10 is present in an amount of from 30% to 60% by weight of said liquid vehicle.

Preferably, the liquid vehicle contains less than 10% by weight ethanol.

The composition can further comprise a hydrophilic surfactant, which can be present in an amount of up to 40%, preferably up to 35%, by weight of the liquid vehicle.

In one embodiment of the invention, the lipophilic surfactant is a propylene glycol mono-ester of a fatty acid, preferably lauroglycol.

It has now been found that the stability problem of testosterone undecanoate associated with free fatty acids can be avoided by the use of triglycerides in which the fatty acids are esterified with glycerol to form a neutral compound which is less reactive. Thus, the invention minimizes or excludes the use of free fatty acids in the compositions.

The triglycerides used in the present invention can liberate long chain fatty acids following lipolysis in the gastrointestinal tract. The long chain triglycerides are used to promote lymphatic absorption in a manner similar to the respective fatty acids. See, for example, Henk de Nijs, Acta Pharmaceutica Technologica 33(4) pp.163-168 (1987), which discusses the enhancement of lymphatic absorption by using triglycerides and different absorption mechanisms involved with long and medium chain fatty acids.

In the present invention, the presence of the lipophilic surfactant, and optionally the additional hydrophilic surfactant, enhances the solubility of testosterone undecanoate in the liquid triglyceride carrier while maintaining the stability of the composition.

Suitable long chain fatty acid (C₁₄-C₂₂) triglycerides for use in the invention include, but are not limited to, arachis oil, soya bean oil, castor oil, corn oil, safflower oil, olive oil, apricot kernel oil, sesame oil, cotton seed oil, sunflower seed oil, palm oil and rapeseed oil.

Suitable lipophilic surfactants for use in the invention are those having an HLB value of less than 10 (HLB < 10). Lipophilic surfactants having an HLB of less than 10 which can be used include, but are not limited to: mono- and di-glycerides of fatty acids; acetic, succinic, lactic, citric and tartaric esters of mono- and di-glycerides of fatty acids; propylene glycol mono- and di-esters of fatty acids; polyglycerol esters of fatty acids; hydrogenated castor oil ethoxylates; acid and ester ethoxylates; sorbitan esters of fatty acids; unsaturated polyglycolized glycerides, alcohol ethoxylates; and polyoxyethylene-polyoxypropylene co-polymers and block co-polymers.

Examples of mono- and di-glycerides of fatty acids which can be used as the lipophilic surfactant include, for example, glyceryl mono/di-caprylate, glyceryl monodi-caprylate/caprate, glyceryl mono-caprylate, glyceryl mono-stearate, glyceryl mono/di-ricinoleate, glyceryl caprylate/caprate, glyceryl mono-oleate, glyceryl dilaurate and glyceryl monostearate

Acetic, succinic, lactic, citric and/or tartaric esters of mono- and/or diglycerides of fatty acids which can be used as the lipophilic surfactant include distilled acetylated monoglycerides, caprylic/capric diglyceryl succinate, mono/disuccinylated monoglycerides, glyceryl stearate citrate, glyceryl monostearate/citrate/lactate, glyceryl cocate/citrate/lactate.

Propylene glycol mono- and/or di-esters of fatty acids which can be used include, for example, lauroglycol (propylene glycol monolaurate) and propylene glycol dicaprylate/dicaprate

Polyglycerol esters of fatty acids suitable as the lipophilic surfactant include polyglyceryl oleate.

Hydrogenated castor oil ethoxylates having low ethoxylate content and HLB less than 10 can also be used, for example, 5 moles of ethylene oxide reacted with 1 mole of castor oil.

Acid and ester ethoxylates formed by reacting ethylene oxide with fatty acids or glycerol esters of fatty acids which can be used include, polyoxyethylene (4) lauric acid, polyoxyethylene (2) stearic acid, polyoxyethylene (3) stearic acid, glyceryl 12 EO dioleate.

Sorbitan esters of fatty acids suitable for use as the lipophilic surfactant include, for example, sorbitan monolaurate, sorbitan monoleate, sorbitan trioleate, sorbitan tristearate.

Examples of unsaturated polyglycolized glycerides include polyoxyethylated apricot kernel oil, polyoxyethylated corn oil, polyoxyethylated hydrogenated oil.

Alcohol ethoxylates which can be used include polyoxyethylated (3) oleyl ether, polyoxyethylated (2) oleyl ether.

Examples of polyoxyethylene-polyoxypropylene co-polymers and block co-polymers can also be used as the lipophilic surfactant.

The lipophilic surfactant is used in an amount ranging from 30% to 60% by weight, typically from 40% to 60% by weight of the liquid vehicle.

Preferred lipophilic surfactants for use in the liquid vehicle are propylene glycol mono- and/or di-esters of fatty acids. Most preferred is lauroglycol (propylene glycol monolaurate) as the lipophilic surfactant.

In a further embodiment, a pharmaceutically acceptable hydrophilic surfactant having an HLB value greater than 10 (HLB > 10) can be used in addition to the long chain fatty acid triglyceride and lipophilic surfactant ingredients of the present invention. Examples of hydrophilic surfactants which can be used include:
a) Phospholipids, in particular lecithins, preferably soyabean lecithins.
b) Polyoxyethylene sorbitan fatty acid derivatives, for example, polyoxyethylene (20) monolaurate, polyoxyethylene (20) monopalmitate, polyoxyethylene (20) monopalmitate, polyoxyethylene (20) sorbitan monostearate, and polyoxyethylene (20) monooleate
c) Hydrogenated castor oil ethoxylates, for example, polyoxyethylene (35) castor oil, polyoxyethylene (40) hydrogenated castor oil, polyoxyethylene (40) castor oil and polyoxyethylene (60) hydrogenated castor oil.
d) Fatty acid ethoxylates, for example, polyoxyethylene (8) stearate, polyoxyethylene (30) monolaurate, polyoxyethylene (20) stearate, polyoxyethylene (15) oleate.
e) Alcohol ethoxylates, for example, polyoxyethylene (10) oleyl ether, polyoxyethylene (30) oleyl ether, polyoxyethylene (20) C12-C14 fatty ether.
f) Polyoxyethylene-polyoxypropylene co-polymers and block co-polymers.
g) Alkylphenol surfactants, for example, polyoxyethylene (9-10) nonylphenol, polyoxyethylene (9) nonylphenol.
h) Saturated polyglycolized medium chain triglycerides, for example, a combination of glyceryl caprylate and polyethylene glycol (8) caprylate/caprate.

Other conventional ingredients or additives can be used in the compositions in accordance with the invention. For example, anti-oxidants such as d-alpha-tocopherol, BHA, BHT and co-solvents such as ethanol, diethylene glycol monoethylether, plasticizers such as propylene glycol, and the like.

Compositions according to the invention can be prepared using conventional methods such as those described in Lacy et al., Patent Cooperation Treaty WO95/24893 published September 21, 1995. A typical process form preparing carrier systems of the invention begins with weighing out the oil component into a suitable stainless steel vessel and then weighing the lipophilic surfactant and adding it to the container. Mixing of the two liquids is effected by the use of a homogenizing mixer or other high shear device. If the material is solid at room temperature, sufficient heat is applied to ensure fluidity without chemical decomposition. If used, the hydrophilic surfactant is added to the other two components. If a hydrophilic solvent is used it is added last with mixing. The drug is then weighed and added to the combined liquids and mixing continued until either a homogenous solution or suspension is prepared. The formulation is then de-aerated before encapsulation in either soft or hard capsules. In some instances, the fill formulation may be held at elevated temperature using a suitable jacketed vessel to aid processing.

The compositions can be encapsulated in softgel or hard shell capsules. Methods of softgel encapsulation are taught in Theory and Practice of Industrial Pharmacy (Lachman & Leiberman, 2^{nd} Edition, publ. Henry Kimpton Publishers, London). Methods of liquid-fill hardshell encapsulation are disclosed in Hardcapsules - Development and Technology, edited by K. Ridgeway, (published by Pharmaceutical Press) (1987).

Embodiments of the invention will now be described by way of example only.

### EXAMPLES:

### Examples 1A through 14A

### Preparation of Liquid Vehicle Formulations

Fourteen liquid vehicle formulations were prepared as measured in parts by weight and contained the following respective ingredients:

| Example: | Ingredients: | % (w/w): |
|---|---|---|
| 1 | Soya Bean oil | 15 |
| | Lauroglycol | 60 |
| | Polyoxyethylene (20) monopalmitate | 25 |
| | | |
| 2 | Arachis oil | 25 |
| | Lauroglycol | 40 |
| | Polyoxyethylene (20) monopalmitate | 35 |
| | | |
| 3 | Arachis oil | 40 |
| | Lauroglycol | 60 |
| | | |
| 4 | Arachis oil | 40 |
| | Glyceryl mono-oleate | 60 |
| | | |
| 5 | Castor oil | 60 |
| | Lauroglycol | 40 |
| | | |
| 6 | Soya Bean oil | 50 |
| | Lauroglycol | 50 |
| | | |
| 7 | Soya Bean oil | 20 |
| | Glyceryl mono/di-caprylate | 50 |
| | Glyceryl caprylate and polyethylene | |
| | glycol (8) caprylate/caprate | 30 |
| | | |
| 8 | Castor oil | 60 |
| | Glyceryl mono/di-caprylate | 40 |
| | | |
| 9 | Castor oil | 40 |
| | Lauroglycol | 55 |
| | Ethanol | 5 |
| | | |
| 10 | Castor oil | 30 |
| | Glyceryl mono/di-caprylate | 40 |
| | Polyoxyethylene (35) castor oil | 30 |
| | | |
| 11 | Castor oil | 50 |
| | Lauroglycol | 30 |
| | Glyceryl caprylate and polyethylene | |
| | glycol (8) caprylate/caprate | 20 |
| | | |
| 12 | Arachis oil | 40 |
| | Glyceryl mono-oleate | 30 |
| | Polyoxyethylene (35) castor oil | 25 |
| | Ethanol | 5 |
| | | |
| 13 | Arachis oil | 25 |
| | Lauroglycol | 60 |
| | Glyceryl caprylate and polyethylene | |
| | glycol (8) caprylate/caprate | 15 |
| | | |
| 14 | Arachis oil | 60 |
| | Glyceryl mono/di-caprylate | 40 |

### Examples 1B through 14B

### Preparation of Pharmaceutical Compositions containing Liquid Vehicle Formulations

Compositions containing an active ingredient and liquid vehicle were prepared by mixing 88 parts by weight of each liquid vehicle formulation as prepared in Examples 1A through 14A together with 12 parts by weight of testosterone undecanoate (TU). The resulting composition contained testosterone undecanoate in stable form which was suitable for encapsulation in the preparation of softgel capsules.

### EXAMPLE 15

### Comparative Stability Test of Testosterone Undecanoate (TU)

Accelerated stability trials were conducted using pharmaceutical compositions containing testosterone undecanoate as the active ingredient in samples using the liquid vehicle according to the invention and a liquid vehicle control sample.

Five samples containing testosterone undecanoate were prepared in accordance with the formulations depicted in Examples 1B through 5B. The control sample containing testosterone undecanoate in combination with oleic acid was prepared. All six samples were then stored for a period of three months.

Following the three month period, each of the resulting samples was analyzed for testosterone undecanoate content. The five samples containing composition prepared according to the invention were found to contain at least 86% and even at least 90 % of the active ingredient, whereas the control sample was found to contain less than 70% of the original testerone undecanoate content.

In conclusion, the data demonstrates that the samples prepared according to the invention exhibited an increased longevity of the testosterone undecanoate in storage when compared to the control sample containing the active ingedient with oleic acid.

### Industrial Applicability

The compositions of the invention enhance the solubility of testosterone undecanoate and improve the storage stability thereof, and can be advantageously used in soft gel and hard shell capsular formulations.

## Claims

1. A liquid pharmaceutical composition having a drug dissolved in a liquid vehicle, said liquid vehicle comprising:
c) a glyceride of a long chain fatty acid; and
d) a lipophilic surfactant having an HLB of less than 10;
**characterised in that** the drug is testosterone undecanoate, said liquid vehicle contains less than 1% by weight of free fatty acids, and said glyceride of a long chain fatty acid is a triglyceride of a fatty acid having 14 to 22 carbon atoms.

2. The liquid pharmaceutical composition according to claim 1, wherein the triglyceride is present in an amount from 15 % to 70 % by weight in the liquid vehicle, and wherein the lipophilic surfactant is present in an amount from 30% to 60% by weight in the liquid vehicle.

3. The liquid pharmaceutical composition according to claim 1 or 2, wherein the triglyceride is selected from the group consisting of arachis oil, soya bean oil, castor oil, corn oil, safflower oil, olive oil, apricot kernel oil and sesame oil, and combinations thereof.

4. The liquid pharmaceutical composition according to claim 1, 2 or 3, wherein the lipophilic surfactant is selected from the group consisting of: mono- and diglycerides of fatty acids; acetic, succinic, lactic and tartaric esters of mono- and di-glycerides of fatty acids; propylene glycol mono- and di-esters of fatty acids; hydrogenated castor oil ethoxylates; acid and ester ethoxylates; sorbitan esters of fatty acids; unsaturated polyglycolized glycerides; alcohol ethoxylates; and polyoxyethylene-propylene co-polymers and block co-polymers, and combinations thereof.

5. The liquid pharmaceutical composition according to claim 4, wherein the lipophilic surfactant is a propylene glycol mono-ester of a fatty acid.

6. The liquid pharmaceutical composition according to claim 5, wherein the lipophilic surfactant is lauroglycol.

7. The liquid pharmaceutical composition according to any preceding claim, further comprising a hydrophilic surfactant.

8. The liquid pharmaceutical composition according to claim 7, wherein the hydrophilic surfactant is selected from the group consisting of phospholipids, polyoxyethylene sorbitan fatty acid derivatives, hydrogenated castor oil ethoxylates, fatty acid ethoxylates, alcohol ethoxylates, polyoxyethylene-polyoxypropylene co-polymers and block co-polymers, alkylphenol surfactants and polyglycolized medium chain triglycerides, and combinations thereof.

9. The liquid pharmaceutical composition according to any preceding claim, further comprising ethanol.

10. The liquid pharmaceutical composition according to claim 9, which contains less than 15 % by weight ethanol.

11. A liquid pharmaceutical composition for oral administration having testerone undecanoate dissolved in a liquid vehicle, said liquid vehicle comprising or consisting of:
a) a triglyceride of a long chain fatty acid having from 14 to 22 carbon atoms; and
b) lauroglycol.

12. A soft gel capsule containing the liquid pharmaceutical composition according to any preceding claim.

13. A hard shell capsule containing the liquid pharmaceutical composition according to any one of claims 1 to 11.

## Patentansprüche

1. Flüssige pharmazeutische Zusammensetzung mit einem Arzneimittel, das in einem flüssigen Vehikel aufgelöst ist, wobei das flüssige Vehikel Folgendes umfasst:
c) Ein Glycerid einer langkettigen Fettsäure; und
d) ein lipophiles Tensid mit einem HLB von weniger als 10;
**dadurch gekennzeichnet, dass** das Arzneimittel Testosteronundecanoat darstellt, das flüssige Vehikel weniger als 1 Gew.-% freie Fettsäuren enthält und das Glycerid aus einer langkettigen Fettsäure ein Triglycerid aus einer Fettsäure mit 14 bis 22 Kohlenstoffatomen darstellt.

2. Flüssige pharmazeutische Zusammensetzung nach Anspruch 1, worin das Triglycerid in einer Menge von 15 Gew.-% bis 70 Gew.-% im flüssigen Vehikel vorliegt und worin das lipophile Tensid in einer Menge von 30 Gew.-% bis 60 Gew.-% im flüssigen Vehikel vorliegt.

3. Flüssige pharmazeutische Zusammensetzung nach Anspruch 1 oder 2, worin das Triglycerid aus der Gruppe ausgewählt ist, bestehend aus Erdnussöl, Sojabohnenöl, Castoröl, Maisöl, Safloröl, Olivenöl, Aprikosenkernöl und Sesamöl und Kombinationen davon.

4. Flüssige pharmazeutische Zusammensetzung nach Anspruch 1, 2 oder 3, worin das lipophile Tensid aus der Gruppe ausgewählt ist, bestehend aus: Mono- und Diglyceriden von Fettsäuren, Essig-, Bernstein-, Milch- und Weinsäureestern von Mono- und Diglyceriden von Fettsäuren; Propylenglykolmono- und -diestern von Fettsäuren; hydrierten Castoröl-Ethoxylaten; Säure- und Esterethoxylaten; Sorbitanestem von Fettsäuren; ungesättigten polyglykolisierten Glyceriden; Alkohol-Ethoxylaten; und Polyoxyethylen-Propylen-Copolymeren und -Blockcopolymeren und Kombinationen davon.

5. Flüssige pharmazeutische Zusammensetzung nach Anspruch 4, worin das lipophile Tensid ein Propylenglykolmonoester einer Fettsäure darstellt.

6. Flüssige pharmazeutische Zusammensetzung nach Anspruch 5, worin das lipophile Tensid Lauroglycol darstellt.

7. Flüssige pharmazeutische Zusammensetzung nach einem der vorangehenden Ansprüche, die weiter ein hydrophiles Tensid umfasst.

8. Flüssige pharmazeutische Zusammensetzung nach Anspruch 7, worin das hydrophile Tensid aus der Gruppe ausgewählt ist, bestehend aus Phospholipiden, Polyoxyethylen-Sorbitan-Fettsäurederivaten, hydrierten Castoröl-Ethoxylaten, Fettsäure-Ethoxylaten, Alkohol-Ethoxylaten, Polyoxyethylen-Polyoxypropylen-Copolymeren und -Blockcopolymeren, Alkylphenol-Tensiden und polyglykolisierten mittelkettigen Triglyceriden und Kombinationen davon.

9. Flüssige pharmazeutische Zusammensetzung nach einem der vorangehenden Ansprüche, weiter umfassend Ethanol.

10. Flüssige pharmazeutische Zusammensetzung nach Anspruch 9, die weniger als 15 Gew.-% Ethanol enthält.

11. Flüssige pharmazeutische Zusammensetzung zur oralen Verabreichung mit Testosteronundecanoat aufgelöst in einem flüssigen Vehikel, wobei das flüssige Vehikel Folgendes umfasst oder besteht aus:
a) Einem Triglycerid aus einer langkettigen Fettsäure mit von 14 bis 22 Kohlenstoffatomen; und
b) Lauroglykol.

12. Weichkapsel enthaltend die flüssige pharmazeutische Zusammensetzung nach einem der vorangehenden Ansprüche.

13. Hartkapsel enthaltend die flüssige pharmazeutische Zusammensetzung nach einem der Ansprüche 1 bis 11.

## Revendications

1. Composition pharmaceutique liquide contenant une substance médicamenteuse dissoute dans un véhicule liquide, ledit véhicule comprenant:
c) un glycéride d'un acide gras à longue chaîne; et
d) un surfactant lipophile dont la valeur de HLB est inférieure à 10;
**caractérisée en ce que** la substance médicamenteuse est l'undécanoate de testostérone, ledit véhicule liquide contient moins de 1 % en poids d'acides gras libres et ledit glycéride d'un acide gras à longue chaîne est un triglycéride d'un acide gras comportant de 14 à 22 atomes de carbone.

2. Composition pharmaceutique liquide selon la revendication 1, dans laquelle le triglycéride est présent en une quantité comprise entre 15% et 70% en poids dans le véhicule liquide et le surfactant lipophile à une quantité comprise entre 30% et 60% en poids dans le véhicule liquide.

3. Composition pharmaceutique liquide selon la revendication 1 ou 2, dans laquelle le triglycéride est sélectionné parmi le groupe consistant en l'huile d'arachide, l'huile de soja, l'huile de ricin, l'huile de maïs, l'huile de carthame, l'huile d'olive, l'huile de noyaux d'abricots et l'huile de sésame et des combinaisons de celles-ci.

4. Composition pharmaceutique liquide selon la revendication 1, 2 ou 3, dans laquelle le surfactant lipophile est sélectionné parmi le groupe consistant en des mono- et diglycérides d'acides gras; des esters acétiques, succiniques, lactiques et tartariques de mono- et diglycérides d'acides gras; des mono- et diesters de propylèneglycol d'acides gras; des éthoxylates d'huile de ricin hydrogénée; des éthoxylates d'esters d'acides; des esters de sorbitan d'acides gras; des glycérides insaturés polyglycolysés; des éthoxylates d'alcool; et des copolymères et des polymères à blocs polyoxyéthylène-propylène, et des combinaisons de ceux-ci.

5. Composition pharmaceutique liquide selon la revendication 4, dans laquelle le surfactant lipophile est le monoester de propylèneglycol d'un acide gras.

6. Composition pharmaceutique liquide selon la revendication 5, dans laquelle le surfactant lipophile est le lauroglycol.

7. Composition pharmaceutique liquide selon l'une quelconque des revendications précédentes, qui comprend également un surfactant hydrophile.

8. Composition pharmaceutique liquide selon la revendication 7, dans laquelle le surfactant hydrophile est sélectionné parmi le groupe consistant en des phospholipides; des dérivés de polyoxyéthylène sorbitan d'acides gras; des éthoxylates d'huile de ricin hydrogénée; des éthoxylates d'acides gras; des éthoxylates d'alcool; des copolymères et des polymères à blocs polyoxyéthylène-propylène; des surfactants alkylphénoliques; et des triglycérides polyglycolysés à chaîne moyenne, et des combinaisons de ceux-ci.

9. Composition pharmaceutique liquide selon l'une quelconque des revendications précédentes, qui contient également de l'éthanol.

10. Composition pharmaceutique liquide selon la revendication 9, qui contient moins de 15% en poids d'éthanol.

11. Composition pharmaceutique liquide destinée à être administrée par voie orale qui contient de l'undécanoate de testostérone dissous dans un véhicule liquide, ledit véhicule liquide comprenant ou consistant en:
a) un triglycéride d'un acide gras à longue chaîne à 14 a 22 atomes de carbone; et
b) du lauroglycol.

12. Gélule de gélatine molle contenant la composition pharmaceutique liquide selon l'une quelconque des revendications précédentes.

13. Gélule de gélatine dure contenant la composition pharmaceutique liquide selon l'une quelconque des revendications 1 à 11.
